# EUROPEAN PATENT APPLICATION

(11) **EP 2 042 164 A1**
(43) Date of publication of application: **01.04.2009**
(21) Application number: 07845214.1
(22) Date of filing: 22.06.2007
(51) Int. Cl.: A61K 9/32, A61K 9/16, A61K 9/20, A61K 9/34, A61K 9/36, A61K 31/10, A61K 47/10, A61K 47/26, A61K 47/32, A61K 47/36, A61K 47/38, A61P 29/00

(54) **STABILIZED SOLID PREPARATION**

(30) Priority: 23.06.2006 JP 2006174119
(71) Applicant: Takeda Pharmaceutical Company Limited, Osaka-shi, Osaka 541-0045 (JP)
(72) Inventor: OHMORI, Shinji, Osaka-shi, Osaka 532-8686 (JP); MATSUOKA, Midori, Osaka-shi, Osaka 532-8686 (JP)
(74) Representative: Weber, Thomas
(86) International application number: PCT/JP2007/062592
(87) International publication number: WO 2007/148786

(57) **Abstract**

A stabilized solid preparation having stabilized drug content and stabilized appearance, comprising a main ingredient layer and a coating layer formed on the main ingredient layer, wherein the main ingredient layer comprises a granule or a fine particle comprising a substance capable of being unstabilized when losing water of crystallization and a water-soluble polymer and the coating layer comprises a water-soluble polymer and a sugar.

## Description

### Technical Field

The present invention relates to a solid preparation containing a drug that is destabilized when water of crystallization is lost, in particular, stabilization of the content of the drug in the solid preparation and stabilization of the appearance of the solid preparation.

### Background Art

There are a variety of mechanisms for destabilization of drugs. As one of them, the presence of drugs that are destabilized when water of crystallization present in the drugs is lost is known. However, a general method for stabilizing such drugs has not been established. At present, a stabilization method suitable for a drug is being sought. For example, it has been known that sodium azulenesulfonate is present as a hemi-hydrate or a monohydrate, and looses water of crystallization due to heat or the like to be destabilized (Nonpatent Document 1). As methods for stabilizing sodium azulenesulfonate, for example, a method comprising spray drying or lyophilization together with a polymer compound (Patent Document 1 and Patent Document 2), and a stabilization method comprising a treatment with a twin-screw extruder (Nonpatent Document 1) have been proposed. However, these methods need special apparatuses for producing a solid preparation or troublesome procedures, and therefore, it is difficult to generally and industrially use these methods to produce a solid preparation of a drug that is destabilized when water of crystallization is lost. For this reason, stabilization methods in the category of production methods of conventional solid preparations (e.g. a method comprising producing a granule for tableting using a fluidized bed granulator, compressing the granule to obtain a plain tablet, and then coating the plain tablet to obtain a coated tablet, etc.) have been desired.

In the case of production of a combination preparation containing other drugs in addition to a drug that is destabilized when water of crystallization is lost for the purpose of enhancing the therapeutic effect, if the above-described method comprising spray drying or lyophilization together with a polymer compound or using a twin-screw extruder is used, the other drugs may be degraded and discolored, and thus, a stable preparation may not be produced. Among vitamins, in particular, vitamin C is easily colored due to heat in the presence of a metal or the like, and vitamin E is a low melting point compound and therefore, is easily melt and softened due to heat to be changed in appearance. For this reason, in the case of a combination preparation containing a drug that is destabilized when water of crystallization is lost in combination with vitamins, particularly vitamin C or vitamin E, even if the content of the drug that is destabilized when water of crystallization is lost can be attained, the solid preparation is changed in appearance, thereby destabilization of appearance may be led.
Patent Document 1: JP-A 1-50225
Patent Document 2: JP-A 1-50226
Nonpatent Document 1: K. Nakamichi et al., Stabilization of sodium guaniazulene sulfonate in granules for tableting prepared using a twin-screw extruder, Eur. J. Pharm. Biopharm., 56, 347-3534 (2003)

### Disclosure of Invention

### Problems to be Solved by the Invention

An object of the present invention is to provide a solid preparation having not only the stabilized content of a drug that is destabilized when water of crystallization is lost, but also the stabilized appearance of the solid preparation by using a water-soluble polymer and further a sugar and by using one or more selected from a fluidized bed granulator, a tumbling fluidized bed granulator, a centrifugal fluidizing granulator, an extrusion granulator and an agitation granulator which are used in production of conventional solid preparations.

### Means to Solving Problems

In order to attain the aforementioned object, the present inventors intensively studied and, as a result, found that a solid preparation having not only the stabilized content of a drug that is destabilized when water of crystallization is lost but also the stabilized appearance of the solid preparation could be produced by using a water-soluble polymer and further a sugar.

In the present invention, fine particles or granules containing a main ingredient (hereinafter, these are collectively referred to as "granules") are prepared by using one or occasionally two or more apparatuses selected from a fluidized bed granulator, a tumbling fluidized bed granulator, a centrifugal fluidizing granulator, an extrusion granulator and an agitation granulator which are used in production of conventional solid preparations, and then, the granules or plain tablets obtained by compressing the granules are subjected to coating, thereby a solid preparation such as a granule, a tablet, or a capsule which is filled with the granule or the tablet can be provided.

That is, the present invention provides:
(1) a stabilized solid preparation comprising a main ingredient layer and a coating layer formed on the main ingredient layer, wherein the main ingredient layer contains granules or fine particles containing a drug that is destabilized when water of crystallization is lost and a water-soluble polymer, and the coating layer contains a water-soluble polymer and a sugar;
(2) the solid preparation according to the above (1), which further comprises an intermediate film layer containing a water-soluble polymer between the main ingredient layer and the coating layer;
(3) the solid preparation according to the above (1) or (2), wherein the granules or fine particles further contain a sugar;
(4) the solid preparation according to any one of the above (1) to (3), wherein the drug that is destabilized when water of crystallization is lost has a solubility of 0.1 w/w% or more in water at 20°C;
(5) the solid preparation according to the above (4), wherein the drug that is destabilized when water of crystallization is lost is sodium azulenesulfonate;
(6) the solid preparation according to any one of the above (1) to (3), wherein the water-soluble polymer is one or more selected from the group consisting of hydroxypropylcellulose, hydroxypropylmethylcellulose, povidone, copolyvidone, polyvinyl alcohol, gum arabic and pullulan;
(7) the solid preparation according to any one of the above (1) to (3), wherein the sugar is one or more selected from the group consisting of erythritol, maltitol, powdered hydrogenated maltose starch syrup, mannitol, purified white sugar, white sugar, trehalose, sorbitol and xylitol;
(8) the solid preparation according to any one of the above (1) to (3), wherein the content of the drug that is destabilized when water of crystallization is lost is 10% by weight or less of the whole preparation;
(9) the solid preparation according to any one of the above (1) to (3), which contains 1 to 100 parts by weight of the water-soluble polymer to 1 part by weight of the drug that is destabilized when water of crystallization is lost;
(10) the solid preparation according to any one of the above (1) to (3), which contains 1 to 1000 parts by weight of the sugar to 1 part by weight of the drug that is destabilized when water of crystallization is lost;
(11) the solid preparation according to any one of the above (1) to (3), which is a tablet or a granule, or a capsule containing the tablet or the granule;
(12) a process for producing the solid preparation according to the above (1) or (2), which comprises preparing granules or fine particles by using a solution of a drug that is destabilized when water of crystallization is lost and a water-soluble polymer, and then using the granules or fine particles; and
(13) a process for producing the solid preparation according to the above (3), which comprises preparing granules or fine particles by using a solution of a drug that is destabilized when water of crystallization is lost, a water-soluble polymer and a sugar, and then using the granules or fine particles.

### Effects of the Invention

According to the present invention, a solid preparation having not only the stabilized content of a drug that is destabilized when water of crystallization is lost but also the stabilized appearance of the solid preparation can be provided by using a water-soluble polymer and further a sugar and by using one or more selected from a fluidized bed granulator, a tumbling fluidized bed granulator, a centrifugal fluidizing granulator, an extrusion granulator and a stirring granulator which are used in production of conventional solid preparations.

### Best Mode for Carrying Out the Invention

The solid preparation of the present invention is a stabilized solid preparation comprising a main ingredient layer which contains granules or fine particles containing a drug that is destabilized when water of crystallization is lost and a water-soluble polymer, and a coating layer formed on the main ingredient layer which contains a water-soluble polymer and a sugar. The main ingredient layer of the solid preparation may further contain a sugar. The solid preparation may comprise an intermediate film layer between the main ingredient layer and the coating layer. Herein, the phrase "destabilized when water of crystallization is lost" means that the crystal structure of a drug is changed, destabilized or degraded because of loss of crystallization water present in the drug. Thus, the term "stabilized" means that a change in the active ingredient content or the appearance of a solid preparation is suppressed by suppressing elimination of water of crystallization from a drug that is destabilized when water of crystallization is lost.

Examples of the drug that is destabilized when water of crystallization is lost, in the present invention, include sodium azulenesulfonate, sodium prasterone sulfate, ampicillin trihydrate, cefixime trihydrate and the like. From a view point of formulation, it is preferable that the drug that is destabilized when water of crystallization is lost has a solubility of 0.1 w/w% or more, preferably 1 w/w% or more in water at 20°C. For example, sodium azulenesulfonate is preferable.

The content of the drug that is destabilized when water of crystallization is lost in the.solid preparation of the present invention is 10% by weight or less, preferably 5% by weight or less of the whole solid preparation, from a viewpoint of the size and the amount of a solid preparation intended to be ingested.

The main ingredient layer of the solid preparation of the present invention may contain other active ingredients in addition to the drug that is destabilized when water of crystallization is lost. Examples of such other active ingredients include, but limited to, vitamins, antacids, stomachics, digesters, antidiarrheics, analgesic antispasmodics, mucosal repairing agents and the like.

Examples of the vitamins include vitamin C such as ascorbic acid, potassium ascorbate and sodium ascorbate, vitamin E such as dl-α-tocopherol calcium succinate, d-α-tocopherol succinate and d-α-tocopherol acetate, L-cysteine, vitamin B1 (thiamine hydrochloride, thiamine nitrate, fursultiamine hydrochloride, dicethiamine hydrochloride, octothiamine, cycothiamine, bisibuthiamine, bisbenthiamine, benfothiamine), vitamin B2 (riboflavin, riboflavin phosphate, riboflavin butyrate), vitamin B6 (pyridoxine hydrochloride, pyridoxal phosphate), vitamin B12 (cyanocobalamine, hydroxocobalamine acetate, mecobalamine), calcium pantothenate, calcium pantothenate type S, nicotinic acid, nicotinic acid amide, gamma oryzanol, orotic acid, glucurolactone, glucuronic acid amide, coix seed, hesperidin, biotin, sodium chondroitin sulfate and the like.

Examples of the antacids include dry aluminum hydroxide gel, magnesium aluminate silicate, magnesium silicate, synthetic hydrotalcite, magnesium oxide, alumina magnesium hydroxide, aluminum hydroxide gel, aluminum hydroxide/sodium hydrogen carbonate coprecipitated product, aluminum hydroxide/magnesium carbonate mixed dry gel, aluminum hydroxide/magnesium carbonate/calcium carbonate coprecipitated product, magnesium hydroxide, sodium hydrogen carbonate, magnesium carbonate, precipitated calcium carbonate, magnesium aluminometasilicate, anhydrous calcium hydrogen phosphate, calcium hydrogen phosphate, aminoacetic acid, dihydroxyaluminum aminoacetate, scopolia extract and the like.

Examples of the stomachics include aloe, fennel, turmeric, fleawort, coptis rhizome, processed garlic, red ginseng, magnolia bark, ginger, swertia herb, cinnamon, rhubarb, panax Japonicus rhizome, citrus unshiu peel, spruce, picrasma wood, carrot, mentha herb, hop, fennel oil, cinnamon oil, ginger oil, spruce oil, mentha oil, lemon oil, 1-menthol, betaine hydrochloride, carnitine chloride, dry yeast and the like.

Examples of the digesters include starch digesting enzyme, protein digesting enzyme, fat digesting enzyme, cellulose digesting enzyme, ursodesoxycholic acid, bile powder and the like.

Examples of the antidiarrheics include acrinol, berberine chloride, guaiacol, creosote, bismuth subsalicylate, bismuth subnitrate, bismuth subcarbonate, bismuth subgallate, tannic acid, kaolin, pectin, medical carbon, calcium lactate, precipitated calcium carbonate, calcium hydrogen phosphate and the like.

Examples of the analgesic antispasmodics include papaverine hydrochloride, ethyl aminobenzoate and the like.

Examples of the mucosal repairing agents include aldioxa, L-glutamine, potassium copper chlorophyllin, sodium copper chlorophyllin, methyl methionine sulfonium chloride, dimethylpolysiloxane and the like.

The water-soluble polymer that can be used in the present invention is a polymer having a solubility in water at 20°C of 1 w/w% or more, preferably 3 w/w% or more, further preferably 5 w/w% or more. Specifically, preferable examples of the water-soluble polymer include one or more selected from hydroxypropylcellulose, hydroxypropylmethylcellulose, povidone, copolyvidone, polyvinyl alcohol, gum arabic, and pullulan.

The amount of the water-soluble polymer to be incorporated in the solid preparation is 1 to 100 parts by weight, preferably 1 to 50 parts by weight to 1 part by weight of the drug that is destabilized when water of crystallization is lost.

Examples of the sugar that can be used in the present invention include erythritol, maltitol, powdered hydrogenated maltose starch syrup, mannitol, purified white sugar, white sugar, trehalose, sorbitol, xylitol, lactose, reduced maltose syrup, glucose, maltose, lactitol and the like. Among them, it is preferable that any one or more selected from erythritol, maltitol, powdered hydrogenated maltose starch syrup, mannitol, purified white sugar, white sugar, trehalose, sorbitol, xylitol and lactose are used as the sugar.

The amount of the sugar to be incorporated in the solid preparation is 1 to 1000 parts by weight, preferably 1 to 500 parts by weight to 1 part by weight of the drug that is destabilized when water of crystallization is lost.

The solid preparation of the present invention may be, for example, in the form of a granule (including a coated granule), a tablet (e.g. a plain tablet, a sugar-coated tablet, a thin layer sugar-coated tablet, a film-coated tablet, a chewable tablet, an oral rapid disintegrating tablet, a laminated tablet, a dry-coated tablet, a sustained-release tablet), a capsule (a hard capsule, a soft capsule), a powdery inhaler, and the like.

The solid preparation of the present invention can be produced using a pharmacologically acceptable carrier or the like which is conventionally used by a conventional method. Particularly, the solid preparation of the present invention can be produced by using one or more selected from a fluidized bed granulator, a tumbling fluidized bed granulator, a centrifugal fluidizing granulator, an extrusion granulator and an agitation granulator which are conventionally used. In other words, the solid preparation of the present invention can be produced by producing a core grain (a granule, a plain tablet or the like), which corresponds to the main ingredient layer of the solid preparation of the present invention, using an active ingredient such as the drug that is destabilized when water of crystallization is lost and an additive such as a carrier, and then coating the core grain with a coating agent. When the granule or tablet thus obtained is small size, the granules or tablets may be filled into a capsule to obtain a capsule preparation.

An example of a method for producing the core grain includes a method which comprises preparing granules or fine particles by using a solution of an active ingredient such as the drug that is destabilized when water of crystallization is lost, the water-soluble polymer and the sugar, mixing the resulting granules or fine particles with additives, and then compressing the mixture to obtain a plain tablet. A further example of a method for producing the core grain includes a method which comprises preparing a solution of the drug, adding the water-soluble polymer (and optionally the sugar) into the solution, and then coating an excipient such as a sugar, celluloses or an inorganic substance, or a core grain or a core tablet such as a granule, a fine particle or a plain tablet which does not contain the drug with the resulting solution.

Examples of the pharmacologically acceptable carrier used in producing the solid preparation of the present invention include various organic or inorganic carrier substances which are conventionally used as pharmaceutical materials, such as excipients, binders, disintegrants, lubricants, glidents, and surfactants. Further, additives such as antioxidants, corrigents, colorants, and flavors may be used in producing the solid preparation of the present invention, if necessary.

Examples of the excipients include erythritol, maltitol, powdered hydrogenated maltose starch syrup, mannitol, purified white sugar, white sugar, trehalose, sorbitol, xylitol, lactose, reduced maltose syrup, glucose, maltose, lactitol, corn starch, microcrystalline cellulose, powdery cellulose, calcium monohydrogen phosphate, calcium hydrogen phosphate, anhydrous calcium hydrogen phosphate, calcium lactate, precipitated calcium carbonate and the like.

Examples of the binders include hydroxypropylcellulose, gelatinized starch, sucrose, gelatin, powdered acacia, methylcellulose, hydroxypropylmethylcellulose, carboxymethylcellulose, carboxymethylcellulose sodium, polyvinylpyrrolidone, polyvinyl alcohol, microcrystalline cellulose, dextrin, pullulan and the like.

Examples of the disintegrants include croscarmellose sodium, low-substituted hydroxypropylcellulose, carmellose calcium, corn starch, carboxymethyl starch sodium, hydroxypropyl starch, partially gelatinized starch, crospovidone and the like.

Examples of the lubricants include magnesium stearate, calcium stearate, sucrose fatty acid ester, talc, Macrogol 6000 and the like.

Examples of the glidents include light anhydrous silicic acid, hydrous silicon dioxide, kaolin and the like.

Examples of the surfactants include polysorbate (e.g. polysorbate 80), polyoxyethylene/polyoxypropylene copolymer, and sodium lauryl sulfate.

Examples of the antioxidants include sodium ascorbate, L-cysteine, and sodium sulfite.

Examples of the corrigents include citric acid, ascorbic acid, tartaric acid, malic acid, aspartame, acesulfame potassium, thaumatin, saccharine sodium, dipotassium glycyrrhizinate, sodium glutamate, sodium 5'-inosinate, and sodium 5'-guanylate.

Examples of the colorants include riboflavin, vitamin B12, titanium oxide, yellow iron sesquioxide, iron sesquioxide, Food Red No.2, Food Red No.3, Food Red No.102, Food Red No.104, Food Red No.105, Food Red No.106, Food Yellow No.4, Food Yellow No.5, Food Green No.3, Food Blue No.1, Food Blue No.2, sodium copper chlorophyll, copper chlorophyll and the like.

Examples of the flavors include 1-menthol, mentha oil, eucalyptus oil, orange oil, clove oil, terebinthine oil, fennel oil, vanillin and the like.

A material for the coating layer that covers the main ingredient layer in the present invention is not particularly limited, as long as the coating layer comprises a sugar. Examples of such sugar include purified white sugar, white sugar, erythritol, trehalose, maltitol, powdered hydrogenated maltose starch syrup, mannitol, sorbitol, xylitol, lactose and the like. The solid preparation of the present invention is stabilized by being sugar-coated or thin layer sugar-coated with one or more kinds of the above-described sugars (see e.g. JP-A 2002-179559).

An example of a method of coating the main ingredient layer in the present invention includes a method comprising producing a core grain such as a granule, a fine particle or a plain tablet which contains the drug, and then coating the core grain. A further example of a method of coating the main ingredient layer in the present invention includes a method comprising directly coating the drug.

The coating layer containing a sugar used in the present invention is not particularly limited as long as it is a sugar coating with a sugar such as purified white sugar, or a thin layer sugar coating with a sugar such as erythritol. If necessary, a coating solution may contain excipients, binders, lubricants, colorants, flavors and the like, in addition to the sugar used in coating.

Examples of the excipients that may be contained in the coating solution include wheat starch, rice starch, corn starch, potato starch, precipitated calcium carbonate, kaolin, hydrous silicon dioxide, calcium silicate, calcium hydrogen phosphate, anhydrous calcium hydrogen phosphate and the like.

Examples of the binders that may be contained in the coating solution include powdered acacia, hydroxypropylcellulose (HPC), hydroxypropylmethylcellulose (HPMC), methylcellulose, hydroxyethylcellulose, carboxymethylethylcellulose, povidone (PVP), polyvinyl alcohol (PVA), pullulan, dextrin, gelatinized starch, hydroxypropyl starch, tragacanth powder, microcrystalline cellulose, low-substituted hydroxypropylcellulose (L-HPC) and the like.

Examples of the lubricants that may be contained in the coating solution include talc, light anhydrous silicic acid, Macrogol 6000, magnesium stearate, calcium stearate, sucrose fatty acid ester and the like.

Examples of the colorants that may be contained in the coating solution include riboflavin, vitamin B12, titanium oxide, yellow iron sesquioxide, iron sesquioxide, Food Red No.2, Food Red No.3, Food Red No.102, Food Red No.104, Food Red No.105, Food Red No.106, Food Yellow No.4, Food Yellow No.5, Food Green No.3, Food Blue No.1, Food Blue No.2, sodium copper chlorophyll, copper chlorophyll and the like.

Examples of the flavors that may be contained in the coating solution include 1-menthol, mentha oil, eucalyptus oil, orange oil, clove oil, terebinthine oil, fennel oil, vanillin and the like.

The main ingredient may be mixed into the coating layer during coating with the coating layer or during storage under high humidity. For the purpose of preventing such commingling, the solid preparation of the present invention may comprise an intermediate film layer between the main ingredient layer and the coating layer. A material for the intermediate film layer is not limited as long as the above-described purpose is attained. For example, the same coating base as that of the coating layer can be used. It is preferable that a water-soluble polymer is contained in the intermediate film layer.

The fluidized bed granulator, the tumbling fluidized bed granulator, the centrifugal fluidizing granulator, the extrusion granulator and the agitation granulator which are used in the present invention are not limited and, for example, apparatuses of Powrex corporation, Freund Corporation, or Fuji Paudal Co., Ltd. may be used.

### Examples

The following Examples illustrate the present invention in more detail. However, the present invention is not limited to the following Examples.

### Example 1

A mixture of 1800 g of calcium ascorbate, 150 g of d-α-tocopherol succinate, 354 g of microcrystalline cellulose (Ceolus KG802) and 36 g of calcium silicate (Florite RE) was granulated with a fluidized bed granulator (MP-10, Powrex) while a solution of 12 g of sodium azulenesulfonate, 144 g of HPC-L and 240 g of erythritol in 2256 g of purified water was sprayed thereto. The resulting granulated powder was subjected to particle size regulation with a particle size regulator (Powermill, Showa Kikai Kako) to obtain size-regulated powder. In a mixing machine (tumbler mixer (15 L), Showa Kagaku Kikai), 2371.2 g of the size-regulated powder, 624 g of L-cysteine, 145.6 g of microcrystalline cellulose (Ceolus KG802), 156 g of low-substituted hydroxypropylcellulose (L-HPC, LH31) and 31.2 g of magnesium stearate were mixed to obtain mixed powder. The resulting mixed powder was compressed with a rotary tableting machine (Correct 12 HUK, Kikusui Seisakusho Ltd.) using a die of 8.8 mm in diameter and a punch having an R surface of 7 mm in curvature radius to obtain plain tablets having a weight of 320 mg and a thickness of 5.54 mm.

Into a coating machine (Hicoater MINI, Freund), 300 g of the resulting plain tablets were placed, and a coating solution or suspension of 90 g of hydroxypropylmethylcellulose (TC-5MW) and 10 g of talc in 900 g of purified water was sprayed to the plain tablets to obtain undercoated (UC) tablets having a coating of 6 mg per tablet. Subsequently, a coating solution or suspension of 530 g of erythritol, 280 g of talc, 20 g of titanium oxide, 50 g of microcrystalline cellulose (Avicel PH-F20) and 120 g of powdered acacia in 1500 g of purified water was sprayed to the UC tablets to obtain build-up coated (BC) tablets having a coating of 114 mg per tablet. Then, the BC tablets were coated with a coating solution of 360 g of erythritol and 40 g of polyethylene glycol 6000 (PEG6000) in 600 g of purified water to obtain syrup-coated (SC) tablets having a coating of 15 mg per tablet. A polishing solution of carnauba wax, shellac pearl, castor oil, ethanol and n-hexane was poured onto the SC tablets, and then dried to obtain polished thin-layer sugarless coated tablets.

### Example 2

A mixture of 1800 g of calcium ascorbate, 150 g of d-α-tocopherol succinate, 354 g of microcrystalline cellulose (Ceolus KG802) and 36 g of calcium silicate (Florite RE) was granulated with a fluidized bed granulator (MP-10, Powrex) while a solution of 12 g of sodium azulenesulfonate, 144 g of PVPK90 and 240 g of erythritol in 2256 g of purified water was sprayed thereto. The resulting granulated powder was subjected to particle size regulation with a particle size regulator (Powermill, Showa Kikai Kako) to obtain size-regulated powder. In a mixing machine (tumbler mixer (15 L), Showa Kagaku Kikai), 2371.2 g of the size-regulated powder, 624 g of L-cysteine, 145.6 g of microcrystalline cellulose (Ceolus KG802), 156 g of low-substituted hydroxypropylcellulose (L-HPC, LH31) and 31.2 g of magnesium stearate were mixed to obtain mixed powder. The resulting mixed powder was compressed with a rotary tableting machine (Correct 12 HUK, Kikusui Seisakusho Ltd.) using a die of 8.8 mm in diameter and a punch having an R surface of 7 mm in curvature radius to obtain plain tablets having a weight of 320 mg and a thickness of 5.54 mm.

A thin-layer sugarless coating was applied to the resulting plain tablets in the same manner as Example 1 to obtain thin-layer sugarless coated tablets.

### Example 3

A mixture of 1800 g of calcium ascorbate, 150 g of d-α-tocopherol succinate, 354 g of microcrystalline cellulose (Ceolus KG802) and 36 g of calcium silicate (Florite RE) was granulated with a fluidized bed granulator (MP-10, Powrex) while a solution of 12 g of sodium azulenesulfonate and 144 g of HPC-L in 2256 g of purified water was sprayed thereto. The resulting granulated powder was subjected to particle size regulation with a particle size regulator (Powermill, Showa Kikai Kako) to obtain size-regulated powder. In a mixing machine (tumbler mixer (15 L), Showa Kagaku Kikai), 2163.2 g of the size-regulated powder, 624 g of L-cysteine, 145.6 g of microcrystalline cellulose (Ceolus KG802), 156 g of low-substituted hydroxypropylcellulose (L-HPC, LH31) and 31.2 g of magnesium stearate were mixed to obtain mixed powder. The resulting mixed powder was compressed with a rotary tableting machine (Correct 12 HUK, Kikusui Seisakusho Ltd.) using a die of 8.8 mm in diameter and a punch having an R surface of 7 mm in curvature radius to obtain plain tablets having a weight of 300 mg and a thickness of 5.26 mm.

A thin-layer sugarless coating was applied to the resulting plain tablet in the same manner as Example 1 to obtain thin-layer sugarless coated tablets.

### Example 4

A mixture of 1800 g of calcium ascorbate, 150 g of d-α-tocopherol succinate, 354 g of microcrystalline cellulose (Ceolus KG802) and 36 g of calcium silicate (Florite RE) was granulated with a fluidized bed granulator (MP-10, Powrex) while a solution of 12 g of sodium azulenesulfonate and 144 g of PVPK in 2256 g of purified water was sprayed thereto. The resulting granulated powder was subjected to particle size regulation with a particle size regulator (Powermill, Showa Kikai Kako) to obtain size-regulated powder. In a mixing machine (tumbler mixer (15 L), Showa Kagaku Kikai), 2163.2 g of the size-regulated powder, 624 g of L-cysteine, 145.6 g of microcrystalline cellulose (Ceolus KG802), 156 g of low-substituted hydroxypropylcellulose (L-HPC, LH31) and 31.2 g of magnesium stearate were mixed to obtain mixed powder. The resulting mixed powder was compressed with a rotary tableting machine (Correct 12 HUK, Kikusui Seisakusho Ltd.) using a die of 8.8 mm in diameter and a punch having an R surface of 7 mm in curvature radius to obtain plain tablets having a weight of 300 mg and a thickness of 5.29 mm.

A thin-layer sugarless coating was applied to the resulting plain tablet in the same manner as Example 1 to obtain thin-layer sugarless coated tablets.

### Comparative Example 1

A mixture of 12 g of sodium azulenesulfonate, 1800 g of calcium ascorbate, 150 g of d-α-tocopherol succinate, 354 g of microcrystalline cellulose (Ceolus KG802) and 36 g of calcium silicate (Florite RE) was granulated with a fluidized bed granulator (MP-10, Powrex) while a solution of 144 g of HPC-L in 2256 g of purified water was sprayed thereto. The resulting granulated powder was subjected to particle size regulation with a particle size regulator (Powermill, Showa Kikai Kako) to obtain size-regulated powder. In a mixing machine (tumbler mixer (15 L), Showa Kagaku Kikai), 2163.2 g of the size-regulated powder, 624 g of L-cysteine, 145.6 g of microcrystalline cellulose (Ceolus KG802), 156 g of low-substituted hydroxypropylcellulose (L-HPC, LH31) and 31.2 g of magnesium stearate were mixed to obtain mixed powder. The resulting mixed powder was compressed with a rotary tableting machine (Correct 12 HUK, Kikusui Seisakusho Ltd.) using a die of 8.8 mm in diameter and a punch having an R surface of 7 mm in curvature radius to obtain plain tablets having a weight of 300 mg and a thickness of 5.26 mm.

### Comparative Example 2

A mixture of 12 g of sodium azulenesulfonate, 1800 g of calcium ascorbate, 150 g of d-α-tocopherol succinate, 354 g of microcrystalline cellulose (Ceolus KG802) and 36 g of calcium silicate (Florite RE) was granulated with a fluidized bed granulator (MP-10, Powrex) while a solution of 144 g of PVPK90 in 2256 g of purified water was sprayed thereto. The resulting granulated powder was subjected to particle size regulation with a particle size regulator (Powermill, Showa Kikai Kako) to obtain size-regulated powder. In a mixing machine (tumbler mixer (15 L), Showa Kagaku Kikai), 2163.2 g of the size-regulated powder, 624 g of L-cysteine, 145.6 g of microcrystalline cellulose (Ceolus KG802), 156 g of low-substituted hydroxypropylcellulose (L-HPC, LH31) and 31.2 g of magnesium stearate were mixed to obtain mixed powder. The resulting mixed powder was compressed with a rotary tableting machine (Correct 12 HUK, Kikusui Seisakusho Ltd.) using a die of 8.8 mm in diameter and a punch having an R surface of 7 mm in curvature radius to obtain plain tablets having a weight of 300 mg and a thickness of 5.29 mm.

### Stability of Sodium azulenesulfonate content

The tablets of Examples 1 to 4 and Comparative Examples 1 and 2 were stored in a sealed glass bottle at 60°C for 2 weeks. The content of sodium azulenesulfonate in the tablet was measured by a HPLC method. The stability was assessed by a remaining rate of sodium azulenesulfonate.

### Test of Sodium azulenesulfonate content

Twenty tablets were ground with a grinding machine to obtain a powder sample. To the powder sample (one tablet) was added 50 mL of a solvent, and the mixture was subjected to shaking extraction for 30 minutes and then centrifuged at about 9400 x g for 10 minutes. To 5 mL of the resulting supernatant was added 5 mL of an internal standard solution, followed by mixing to obtain a sample solution. The sample solution was subjected to measurement under the following HPLC conditions. As the solvent, methanol/water (6/4) was used. As the internal standard solution, a solution of bis(2-ethylhexyl) phthalate in methanol was used.
HPLC Conditions
Detector: ultraviolet absorptiometer (measuring wavelength: 285 nm)
Column: YMC-Pack AM-3E2 (YMC)
Column temperature: 50°C
Mobile phase: 0.003 mol/L trioctylamine-containing methanol/water (17/3) mixed solution (pH 6.0)
Flow rate: 0.35 mL/min [Table 1] Stability of Sodium azulenesulfonate content (remaining rate (%), stored at 60°C for 2 weeks) Example Example Example Example Comparative Comparative 1 2 3 4 Example 1 Example 2 96.6 98.9 88.1 98.0 54.4 18.7

### Stability of Appearance

Tablets of Examples 1 and 2 and Comparative Examples 1 and 2 were stored in an open container at 40°C and 75% RH for 1 month. A change in the appearance of the tablet was measured with a color computer (S&M Color Computer, Suga Test Instrument Co., Ltd.). The stability was assessed by a color difference (ΔE). In addition, regarding Examples 1 and 2, after open storage at 40°C and 75% RH for 1 month, the coating layer of the tablet was cut off with a cutter, and a change in the appearance of the inside of the tablet was also assessed by a color difference (ΔE).

**[Table 2]**

| Stability of Appearance | | | | |
|---|---|---|---|---|
| (ΔE, stored at 40°C and 75% RH in an open container) | | | | |
| | Example 1 | Example 2 | Comparative Example 1 | Comparative Example 2 |
| Outside of tablet | 2.5 | 2.1 | 14.7 | 21.9 |
| Inside of tablet | 2.7 | 3.2 | - | - |

The tablets of Examples 1 and 2 had not only a stabilized drug content, but also stabilized appearances of both the outside and the inside of the tablet, as compared with the tablets of Comparative Examples 1 and 2.

## Claims

1. A stabilized solid preparation comprising a main ingredient layer and a coating layer formed on the main ingredient layer, wherein the main ingredient layer contains granules or fine particles containing a drug that is destabilized when water of crystallization is lost and a water-soluble polymer, and the coating layer contains a water-soluble polymer and a sugar.

2. The solid preparation according to claim 1, which further comprises an intermediate film layer containing a water-soluble polymer between the main ingredient layer and the coating layer.

3. The solid preparation according to claim 1 or 2, wherein the granules or fine particles further contain a sugar.

4. The solid preparation according to any one of claims 1 to 3, wherein the drug that is destabilized when water of crystallization is lost has a solubility of 0.1 w/w% or more in water at 20°C.

5. The solid preparation according to claim 4, wherein the drug that is destabilized when water of crystallization is lost is sodium azulenesulfonate.

6. The solid preparation according to any one of claims 1 to 3, wherein the water-soluble polymer is one or more selected from the group consisting of hydroxypropylcellulose, hydroxypropylmethylcellulose, povidone, copolyvidone, polyvinyl alcohol, gum arabic and pullulan.

7. The solid preparation according to any one of claims 1 to 3, wherein the sugar is one or more selected from the group consisting of erythritol, maltitol, powdered reduced maltose syrup, mannitol, purified white sugar, white sugar, trehalose, sorbitol and xylitol.

8. The solid preparation according to any one of claims 1 to 3, wherein the content of the drug that is destabilized when water of crystallization is lost is 10% by weight or less of the whole preparation.

9. The solid preparation according to any one of claims 1 to 3, which contains 1 to 100 parts by weight of the water-soluble polymer to 1 part by weight of the drug that is destabilized when water of crystallization is lost.

10. The solid preparation according to any one of claims 1 to 3, which contains 1 to 1000 parts by weight of the sugar to 1 part by weight of the drug that is destabilized when water of crystallization is lost.

11. The solid preparation according to any one of claims 1 to 3, which is a tablet or a granule, or a capsule containing the tablet or the granule.

12. A process for producing the solid preparation according to claim 1 or 2, which comprises preparing granules or fine particles by using a solution of a drug that is destabilized when water of crystallization is lost and a water-soluble polymer, and then using the granules or fine particles.

13. A process for producing the solid preparation according to claim 3, which comprises preparing granules or fine particles by using a solution of a drug that is destabilized when water of crystallization is lost, a water-soluble polymer and a sugar, and then using the granules or fine particles.
